# EUROPEAN PATENT SPECIFICATION

(11) **EP 0 888 366 B1**
(45) Date of publication and mention of the grant of the patent: **09.06.2004**
(21) Application number: 97916793.9
(22) Date of filing: 18.03.1997
(51) Int. Cl.: C07H 1/00, C08B 37/06, A01N 43/04

(54) **MODIFIED PECTIN MATERIAL**
MODIFIZIERTES PEKTINMATERIAL
MATIERE PECTIQUE MODIFIEE

(30) Priority: 21.03.1996 US 13836 P
(43) Date of publication of application: 07.01.1999
(73) Proprietor: Platt, David, Boston, MA 02116 (US)
(72) Inventor: Platt, David, Boston, MA 02116 (US)
(74) Representative: Horner, Martin Grenville
(86) International application number: PCT/US1997/004205
(87) International publication number: WO 1997/034907

(56) References cited:
- WO-A-96/01640
- US-A- 5 547 945
- D. PLATT ET AL.: "Modulation of the Lung Colonization of B16-F1 Melanoma Cells by Citrus Pectin" JOURNAL OF NATIONAL CANCER INSTITUTE, vol. 84, no. 6, 18 March 1992 (1992-03-18), pages 438-442, XP002115764
- CHEMICAL ABSTRACTS, Vol. 108, 1988, (Columbus, Ohio, USA), FRANZ G., "Structure-Activity Relation of Polysaccharides With Antitumor Activity", page 20, Abstract No. 31316n; & FARM. TIJDSCHR. BELG., 1987, 64(4), pages 301-311, (Eng.).

## Description

This invention relates generally to carbohydrates. More specifically, the invention relates to pectin derived materials. Most specifically, the invention relates to a particular group of chemically modified pectins which have been found to have therapeutic utility.

As disclosed in U.S. Patent Application Serial No. 08/024,487 filed March 1, 1993 and entitled "Modified Pectin", certain chemically modified pectins have therapeutic utility in the treatment and prevention of metastatic cancer. These materials are prepared by a general process in which pectin is partially depolymerized by disrupting the rhamnogalcturan backbone thereof, and by breaking the side chains of neutral sugars into smaller units. Typically, the backbone is disrupted under alkaline conditions, and the side chains of neutral sugars are broken up under acidic conditions.

D. Platt et al in "Modulation of the Lung Colonization B16-F1 Melanoma Cells by Citrus Pectin" of the Journal of National Cancer Institute, vol.84, No. 6, 18 March 1992 (1992-03-18), pages 438-442 refers to unbranched pectin material and modified citrus pectins which have a quite different sugar composition to that of the present invention.

In accord with the present invention, particular modified pectins having therapeutic utility and a specific structure have been prepared and identified. The modified pectin material has a shortened rhamnogalacturan backbone with shortened chains of neutral sugars dependent therefrom. In addition, it has been found that in the preferred materials most or all of the methoxyl groups have been removed from the backbone, and the molecular weight of the modified pectin is approximately 10,000.

### Brief Description of the Invention

There is disclosed herein a modified pectin material which comprises a rhamnogalacturan backbone having a repeating sequence of two galacturonic acid units followed by one rhamnose unit. The modified pectin further includes a first and second group of side chains of neutral sugars dependent from the backbone via those rhamnose units which are separated from one another by an intervening sequence comprising two galacturonic units, a rhamnose unit and two more galacturonic acid units. The first group of side chains comprises straight chains of neutral sugars and the second comprises branched chains of neutral sugars. The average molecular weight of the modified pectin is in the range of approximately 5,000 to 100,000, as determined by viscosity measurements. In one preferred group of materials, the average molecular weight is approximately 10,000.

In particular embodiments, the first group of side chains comprises straight chains of 3-8 linked monosaccharide units, and these units may, in some instances, be galactose units. In other particular embodiments, the second group of side chains comprises multiply branched side chains which may be based upon arabinose units. In certain embodiments, multiply branched side chains of the second group comprises 10-40 linked monosaccharide units.

In a particularly preferred class of materials, the side chains of neutral sugars comprise, on a weight basis, at least 6% galactose, and most preferably 10% galactose .

In one specific pectin, the second group of side chains comprises a chain of arabinose units having side chains of arabinose units branching therefrom, and the side chains are terminated by either arabinose, galactose, feruloyl or glucose. In yet other instances, the pectin material can include a third group of side chains of neutral sugars comprising a straight chain of 2-4 monosaccharides joined to the backbone through the galacturonic acid units. One particular pectin material comprises, by weight, approximately 28% galacturonic acid, 39% arabinouronic acid and/or arabinose, 12% rhamnose, 10% galactose and 8% glucose.

### Brief Description of the Drawing

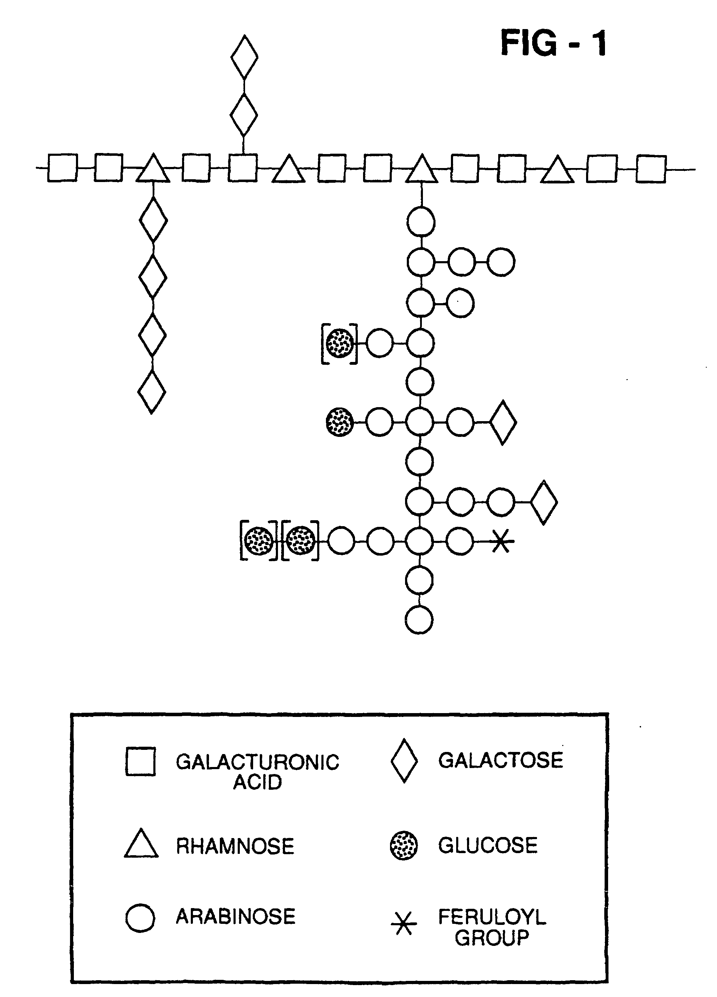

Figure 1 is a schematic depiction of a generalized molecular structure of the modified pectin material of the present invention.

### Detailed Description of the Invention

In accord with the present invention, it has been found that therapeutically useful modified pectin includes a rhamnogalacturan backbone comprising a repeating sequence of two galacturonic acid units followed by one rhamnose unit. Neutral sugar side chains are attached to the backbone through the rhamnose units, and these side chains are attached to those rhamnose units separated by five intervening units, which comprise, in sequence: two galacturonic units, one rhamnose unit and two more galacturonic units. The neutral side chains will include a first group of side chains comprising straight chains of neutral sugars and a second group of side chains comprising branched chains of neutral sugars. In addition, a third group of side chains may also be present, and these will comprise relatively small, straight chains of 2-4 monosaccharides joined to the backbone through the galacturonic acid units. It has been found that therapeutic utility is best when galactose comprises, by weight, at least 6%, and preferably 10%, of the neutral sugars.

The number and identity of the side chains may vary to some degree, and the materials of the present invention will typically have a molecular weight in the range of 5,000 to 100,000; although it has been found that the modified pectin most preferably has a molecular weight in the range of approximately 5,000 to 50,000, and one specific, preferred material has an average molecular weight of approximately 10,000 as determined by viscosity measurements at 25°C in an Ubbelholde No. 1 viscometer, with sodium-hexamethyl phosphate at 20 mm (pH 4.5) and 0.2% EDTA, 0.9% NaCl. It has also been found that when the reaction conditions (typically acid) employed to break the side chains of neutral sugars into smaller units are varied, the nature of the side chains will vary; but, therapeutic utility remains, provided that the particular rhamnogalacturan backbone structure is maintained, and provided that the side chains include galactose. Therefore, while not wishing to be bound by speculation, Applicant presumes that therapeutic utility is strongly dependent upon the configuration of the backbone, and the presence of galactose in the neutral sugars comprising the side chains.

Referring now to Figure 1, there is shown a schematic depiction of a molecule of modified pectin in accord with the present invention. As depicted, the backbone portion comprises a repeating series of two galacturonic acid units followed by a rhamnose unit. Side chains of neutral sugars branch from rhamnose units which are separated by at least five intervening units; although as will be noted, not every rhamnose unit which could carry a side chain has one.

As shown in Figure 1, the modified pectin includes straight side chains such as the chain of four galactose units. The pectin also includes highly branched side chains such as the arabinose based, branched side chain. In general, the structure of the highly branched side chain is less variable than is the structure of the relatively straight chain. Figure 1 depicts a typical structure, characteristic of the branched chain. As will be noted, several of the glucose groups are shown as being in brackets; this indicates that these groups are optional. As mentioned previously, other, relatively small, straight, side chains may also be present, and these chains depend from the galacturonic acid units. One such chain is shown in Figure 1 and includes two galactose units. While unmodified pectins generally include a significant percentage of methoxyl groups and the like; in the preferred material of the present invention most, if not all, of such groups have been removed.

A number of specific structures will fall within the definition of the modified pectin of the present invention.

One such particular material has been found to include, by weight, approximately 28% galacturonic acid, 39% arabinouronic acid and/or arabinose, 12% rhamnose, 10% galactose and 8% glucose, as determined by gas chromatography/mass spectrometry.

### Preparation of the Material

In general, the material of the present invention is prepared by disrupting the rhamnogalaciuran backbone of the pectin, and by breaking down the side chains of neutral sugars therefrom. In this treatment, most of the methoxyl groups are also removed from the backbone. A number of particular procedures may be employed; and in one specific procedure, citrus pectin (70-100 kilodalton 0.5%, from the Sigma Chemical Company of St. Louis, Missouri), which included 10% methoxyl groups, was solubilized and sterilized under UV radiation for 48 hours in distilled water. The total carbohydrate level was determined by the phenol sulfuric acid method. The pH of the solution was increased to 10 with 3N NaoH, maintained for thirty minutes and then decreased to 3.0 with 3N HCl. The solution was maintained at a pH of 3.0 for approximately ten hours and then equilibrated to 6.3. The solution was washed with 70% ethanol and dried with 100% acetone. The material thus produced was analyzed via gas chromatography/mass spectrometry to yield the structural data presented hereinabove.

Other procedures and experimental conditions may be employed to prepare the modified pectin material of the present invention provided that the methodologies depolymerize the backbone and reduce the side chains as detailed above. The material of the present invention has a novel and unique structure characterized by a rhamnogalacturan backbone which is comprised of at least three repeating sequences of two galacturonic acid units followed by one rhamnose unit. In the backbone, these sequences are linearly joined so that the rhamnose unit of one sequence is coupled to the galacturonic acid unit of another. The material further includes two different groups of side chains of neutral sugars dependent from the backbone. The first group of side chains comprise relatively short, straight chains of neutral sugars and the second comprise highly branched chains of neutral sugars. The side chains are joined to the backbone through the rhamnose units, and are spaced apart from one another by five intervening backbone units, namely an intervening sequence of two galacturonic acid units, one rhamnose unit and two more galacturonic acid units. Most preferably, the neutral sugars of the side chains include at least 6%, and most preferably 10%, by weight galactose. The particular configuration of the material of the present invention has been found to provide a therapeutic material having utility in the treatment of metastatic cancers.

In view of the disclosure and teaching presented herein, various modified pectin materials may be prepared. The foregoing examples and discussion are illustrative of particular embodiments of the present invention, but are not meant to be limitations upon the practice thereof.

## Claims

1. A modified pectin material comprising:
a rhamnogalacturan backbone comprising a repeating sequence of two galacturonic acid units followed by one rhamnose unit;
said modified pectin further including a first and a second group of side chains of neutral sugars dependent from said backbone, members of said first and second group of side chains being attached to said backbone through rhamnose units thereof which are separated from one another by an intervening sequence comprising two galacturonic acid units, a rhamnose unit and two galacturonic acid units; said first group of side chains comprising straight chains of neutral sugars and said second group of side chains comprising branched chains of neutral sugars; said modified pectin having an average molecular weight in the range of 5,000 to 100,000.

2. A modified pectin material as in claim 1, wherein members of said first group of side chains are straight chains comprised of 3-8 monosaccharide units.

3. A modified pectin material as in claim 2, wherein said monosaccharide units are galactose units.

4. A modified pectin material as in claim 1, wherein members of said second group of side chains are multiply branched side chains.

5. A modified pectin material as in claim 4, wherein said multiply branched chains are multiply branched chains comprised of arabinose units.

6. A modified pectin material as in claim 4, wherein said multiply branched chain comprises 10-40 monosaccharide units.

7. A modified pectin material as in claim 1, wherein the members of said second group of side chains comprise a chain of arabinose units having side chains of arabinose units branching therefrom, said side chains being terminated by members selected from the group consisting of: arabinose, galactose, feruloyl groups, glucose, and combinations thereof.

8. A modified pectin material as in claim 1, further including a third group of side chains of neutral sugars, members of said third group comprising straight chains of 2-4 monosaccharides which are joined to said backbone through the galacturonic acid units thereof.

9. A modified pectin material as in claim 1, wherein the molecular weight of said second side chain is greater than the molecular weight of said backbone.

10. A modified pectin as in claim 1, comprising approximately 28% galacturonic acid; 39% arabinouronic acid and/or arabinose; 12% rhamnose; 10% galactose and 8% glucose.

11. A modified pectin as in claim 1, wherein the average molecular weight thereof is approximately 10,000.

12. A modified pectin as in claim 1, wherein galactose comprises, by weight, at least 6% of the neutral sugars of said side chains.

13. A modified pectin as in claim 1, wherein galactose comprises, by weight, 10% of the neutral sugars of said side chains.

## Patentansprüche

1. Modifiziertes Pektinmaterial, enthaltend
ein Rhamnogalacturangerüst, das eine sich wiederholende Sequenz aus Galacturonsäureeinheiten, gefolgt von einer Rhamnoseeinheit, aufweist,
wobei das modifizierte Pektin ferner eine erste und eine zweite Gruppe von Seitenketten aus neutralen Zuckern aufweist, die an dem genannten Gerüst hängen, wobei Glieder der ersten und der zweiten Gruppe von Seitenketten an das genannte Gerüst durch daran befindliche Rhamnoseeinheiten gebunden sind, die voneinander durch eine dazwischenliegende Sequenz getrennt sind, die zwei Galacturonsäureeinheiten, eine Rhamnoseeinheit und zwei Galacturonsäureeinheiten aufweist, wobei die erste Gruppe der Seitenketten gerade Ketten aus Zuckern und die zweite Gruppe der Seitenketten verzweigte Ketten aus neutralen Zuckern beinhalten, und wobei das modifizierte Pektin ein Durchschnittsmolekulargewicht im Bereich von 5.000 bis 100.000 aufweist.

2. Modifiziertes Pektinmaterial nach Anspruch 1, worin Glieder der ersten Gruppe der Seitenketten gerade Ketten mit 3 bis 8 Monosaccharideinheiten sind.

3. Modifiziertes Pektinmaterial nach Anspruch 2, worin die Monosaccharideinheiten Galactoseeinheiten sind.

4. Modifiziertes Pektinmaterial nach Anspruch 1, worin Glieder der zweiten Gruppe der Seitenketten mehrfach verzweigte Seitenketten sind.

5. Modifiziertes Pektinmaterial nach Anspruch 4, worin die mehrfach verzweigten Ketten mehrfach verzweigte Ketten mit Arabinoseeinheiten sind.

6. Modifiziertes Pektinmaterial nach Anspruch 4, worin die mehrfach verzweigte Kette 10 bis 40 Monosaccharideinheiten enthält.

7. Modifiziertes Pektinmaterial nach Anspruch 1, worin die Glieder der zweiten Gruppe der Seitenketten eine Kette aus Arabinoseeinheiten mit davon abzweigenden Seitenketten aus Arabinoseeinheiten aufweisen, wobei die genannten Seitenketten durch Glieder beendet sind, die aus Arabinose, Galactose, Feruloylgruppen, Glucose und Kombinationen hiervon ausgewählt sind.

8. Modifiziertes Pektinmaterial nach Anspruch 1, das ferner eine dritte Gruppe von Seitenketten aus neutralen Zuckern aufweist, wobei Glieder dieser dritten Gruppe gerade Ketten aus 2 bis 4 Monosacchariden enthalten, die an das genannte Gerüst über daran befindliche Galacturonsäureeinheiten gebunden sind.

9. Modifiziertes Pektinmaterial nach Anspruch 1, worin das Molekulargewicht der zweiten Seitenkette größer als das Molekulargewicht des genannten Gerüstes ist.

10. Modifiziertes Pektin nach Anspruch 1, enthaltend etwa 28% Galactur+onsäure, 39% Arabinouronsäure und/oder Arabinose, 12% Ramhnose, 10% Galactose und 8% Glucose.

11. Modifiziertes Pektin nach Anspruch 1, worin dessen Durchschnittsmolekulargewicht etwa 10.000 beträgt.

12. Modifiziertes Pektin nach Anspruch 1, worin Galactose mindestens 6%, bezogen auf das Gewicht, neutrale Zucker der genannten Seitenketten enthält.

13. Modifiziertes Pektin nach Anspruch 1, worin Galactose 10%, bezogen auf das Gewicht, der neutralen Zucker der Seitenketten enthält.

## Revendications

1. Matière pectique modifiée comprenant :
un squelette de rhamnogalacturane comprenant une séquence répétitive de deux unités d'acide galacturonique suivies d'une unité de rhamnose ;
ladite matière pectique modifiée comprenant en outre un premier et un second groupe de chaînes latérales de sucres neutres dépendantes dudit squelette, des éléments dudit premier et deuxième groupe de chaînes latérales étant fixés au dit squelette par le biais d'unités de rhamnose de celui-ci, qui sont séparées les unes des autres par une séquence intermédiaire comprenant deux unités d'acide galacturonique une unité rhamnose et deux unités d'acide galacturonique ; ledit premier groupe des chaînes latérales comprenant des chaînes linéaires de sucres neutres et ledit second groupe de chaînes latérales comprenant des chaînes ramifiées de sucres neutres ; ladite pectine modifiée ayant un poids moléculaire moyen dans la plage allant de 5000 à 100 000.

2. Matière pectique modifiée selon la revendication 1, dans laquelle les éléments dudit premier groupe de chaînes latérales sont des chaînes linéaires comprenant de 3 à 8 unités de monosaccharide.

3. Matière pectique modifiée selon la revendication 2, dans laquelle lesdites unités de monosaccharide sont des unités de galactose.

4. Matière pectique modifiée selon la revendication 1, dans laquelle des éléments dudit second groupe de chaînes latérales sont des chaînes latérales à ramifications multiples.

5. Matière pectique modifiée selon la revendication 4, dans laquelle lesdites chaînes à ramifications multiples sont des chaînes à ramifications multiples d'unités d'arabinose.

6. Matière pectique modifiée selon la revendication 4, dans laquelle ladite chaîne à ramifications multiples comprend 10 à 40 unités de monosaccharide.

7. Matière pectique modifiée selon la revendication 1, dans laquelle les éléments dudit second groupe de chaînes latérales comprennent une chaîne d'unités d'arabinose ayant des chaînes latérales d'unités d'arabinose partant de celles-ci, lesdites chaînes latérales étant terminées par des éléments choisis parmi le groupe constitué de : l'arabinose, le galactose, les groupes féruloyle, le glucose et des combinaisons de ceux-ci.

8. Matière pectique modifiée selon la revendication 1, comprenant en outre un troisième groupe de chaînes latérales de sucres neutres, des éléments dudit troisième groupe comprenant des chaînes linéaires de 2 à 4 monosaccharides qui sont jointes au dit squelette par le biais d'unités d'acide galacturonique de celui-ci.

9. Matière pectique modifiée selon la revendication 1, dans laquelle le poids moléculaire de ladite seconde chaîne latérale est supérieur au poids moléculaire dudit squelette.

10. Pectine modifiée selon la revendication 1, comprenant environ 28 % d'acide galacturonique ; 39 % d'acide arabinouronique et/ou d'arabinose ; 12 % de rhamnose, 10 % de galactose et 8 % de glucose.

11. Pectine modifiée selon la revendication 1, dans laquelle le poids moléculaire moyen de celle-ci est d'environ 10 000.

12. Pectine modifiée selon la revendication 1, dans laquelle le galactose comprend, en poids, au moins 6 % des sucres neutres desdites chaînes latérales.

13. Pectine modifiée selon la revendication 1, dans laquelle le galactose comprend, en poids, 10 % des sucres neutres desdites chaînes latérales.
